(19) Europäisches Patentamt
European Patent Office
Office européen des brevets

(11) Numéro de publication: **0 272 181 B1**

(12) **FASCICULE DE BREVET EUROPEEN**

(45) Date de publication de fascicule du brevet: **21.08.91**

(51) Int. Cl.$^5$: **C07C 321/04**

(21) Numéro de dépôt: **87402767.5**

(22) Date de dépôt: **04.12.87**

Le dossier contient des informations techniques présentées postérieurement au dépôt de la demande et ne figurant pas dans le présent fascicule.

# (54) Synthèse de dithiols.

(30) Priorité: **17.12.86 FR 8617640**

(43) Date de publication de la demande: **22.06.88 Bulletin 88/25**

(45) Mention de la délivrance du brevet: **21.08.91 Bulletin 91/34**

(84) Etats contractants désignés: **BE DE ES FR GB IT NL**

(56) Documents cités:
**EP-A- 0 060 754**
**US-A- 4 140 604**

(73) Titulaire: **SOCIETE NATIONALE ELF AQUITAINE (PRODUCTION)**
**Tour Elf, 2, Place de la Coupole, La Défense 6**
**F-92400 Courbevoie(FR)**

(72) Inventeur: **Ollivier, Jean**
**Croix de Buzy**
**F-64260 Arudy(FR)**

(74) Mandataire: **Leboulenger, Jean et al**
**ATOCHEM Département Propriété Industrielle**
**F-92091 Paris la Défense 10 Cédex 42(FR)**

EP 0 272 181 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'invention se rapporte à la production de dithiols par l'action de l'hydrogène sulfuré sur des diènes. Elle s'applique plus particulièrement à la synthèse de dithiols formés à partir de diènes comportant plus de 4 atomes de carbone.

La préparation de mercaptans en général, aliphatiques et aromatiques, a donné lieu à des nombreux travaux, à cause de l'utilité industrielle croissante de ces composés. On connaît notamment le procédé photochimique, qui consiste à faire réagir $H_2S$ avec une oléfine, les réactifs étant irradiés Par l'émission d'une lampe à mercure en présence d'un initiateur tel que, par exemple, une alcoxyacétophénone (brevet US 4 140 604), un mélange de benzophénone ou thiobenzophénone et un phosphite de trialkyle (brevet FR 2 424 907), ou un composé xanthénique seul ou associé à un phosphite ou une phosphine d'alkyle (brevet EP 0 060 754). Ce procédé donne d'excellents résultats avec des monooléfines.

Mais dans les cas de diènes, des réactions secondaires se produisent compromettant le rendement en le dithiol désiré. Il semble que l'interaction du dithiol formé avec le diène lui-même, pour donner des polymères, joue un rôle important. Or,les autres méthodes connues d'obtention de dithiols, à savoir l'action de dihalogénures d'alkyles correspondants sur un sel alcalin de $H_2S$, directement ou par l'intermédiaire d'un bis-thiocyanate, bis-thiouréthane ou bis-thiol-acétate ("Organic Sulfur Compounds" de N. KARASCH, Vol. 1, Pergam. Press. 1961 p. 199-207), comportent plusieurs stades et leurs rendements laissent à désirer. Il y avait donc intérêt à rechercher à appliquer un procédé plus direct et commode, comme celui du brevet français précité, à la fabrication de dithiols.

Une telle recherche a été réussie dans le cadre de la présente invention : celle-ci permet, en effet, d'obtenir des dithiols à partir de diènes non conjugués par l'action directe de l'$H_2S$, avec de bons rendements et pureté, à condition de prendre certaines précautions spéciales, qui ne pouvaient pas être prévues à partir de la technique antérieure. Ainsi, la présente invention permet-elle d'obtenir des dithiols, par la fixation d'un $H_2S$ sur chacune des doubles liaisons d'un diène, avec un minimum d'intervention d'une réaction du diène avec le dithiol formé, et sans polymérisation subséquente.

Le procédé suivant l'invention consiste à faire réagir de l'hydrogène sulfuré sur un ou plusieurs diènes non conjugué (s), en présence d'une lumière de 300 nm à 600 nm, et d'un système catalytique, comprenant un phosphite de trialkyle et un initiateur constitué par une cétone ou thiocétone aromatique, caractérisé en ce que le milieu réactionnel est constitué par un excès de $H_2S$ liquide, tenant en solution ou en dispersion 2,5 à 10% en volume de diène.

Le système catalytique, utilisable dans la présente invention, peut comprendre en tant qu'initiateur une cétone ou thiocétone telle que la benzophénone, la thiobenzophénone ou un composé répondant à la formule :

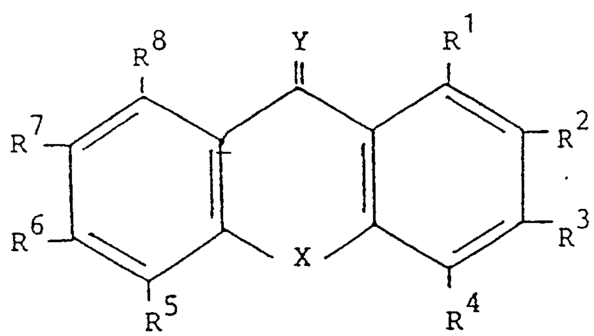

(1)

où Y est O ou S, tandis que X désigne un élément O, S ou Se, un groupe $-CH_2$, $-CH_2CH_2-$, $SO_2$, CO, CS, NR', R' étant un radical hydrocarboné, ou bien un groupement au phosphore tri- ou pentavalent, notamment

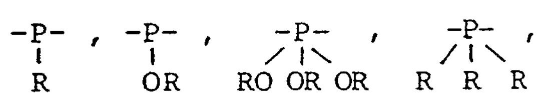

où R désigne un alkyle, un aryle, un H ou un halogène. Les symboles $R^1$ à $R^8$ désignent des atomes ou groupes semblables ou différents, tels que H, halogène, alcoxy, alkyle ou/et aryle.

Il est curieux de constater que le rendement en le dithiol baisse lorsque la concentration en diène, dans

le milieu réactionnel, augmente au-dessus de 10%. Ainsi,des rendements de l'ordre de 50% peuvent encore être obtenus avec des teneurs en diène atteignant environ 40%, mais ces teneurs doivent être dans la marge de 5 à 10% si l'on cherche à obtenir des rendements avoisinant 70%, industriellement intéressants.

Suivant un trait préféré de l'invention, on ne laisse pas s'accumuler plus de 10% en volume de dithiol formé, dans le milieu réactionnel. De préférence cette accumulation est tenue bien au-dessous de 10%. Cela signifie que le dithiol produit doit être retiré de sa solution dans l'$H_2S$ liquide, aussi rapidement que possible, le mieux au fur et à mesure de sa formation. On y parvient aisément en marche continue, par le soutirage continuel du milieu ou de la solution réactionnelle, dont on extrait le dithiol et recycle l'$H_2S$ et le diène non converti.

La cétone et le phosphite sont dissous dans le milieu réactionnel,en général à raison de 0,01 à 0,1 mole/litre chacun.

Le procédé suivant l'invention peut être réalisé aux mêmes températures modérées que le procédé de la technique connue, en particulier celui du brevet français mentionné plus haut. On peut donc travailler à des températures de -10° C à +50° C et de préférence entre -5° C et +15° C, les températures de 0° C à 10° C étant particulièrement favorables pour un compromis entre la cinétique de la réaction et la pression exigée pour le maintien de l'$H_2S$ à l'état liquide.

Il s'en suit que le procédé de l'invention est le plus souvent réalisé sous des pressions d'environ 12 à 25 bars.

Le procédé s'applique en général à des dioléfines pouvant avoir des nombres d'atomes de carbone très différents et des positions variées des doubles liaisons. Un groupe fort attrayant parmi ces diènes comporte 5 à 20 atomes de carbone ; en particulier des dithiols très utiles sont obtenus à partir de diènes en $\alpha$- $\omega$, c'est-à-dire porteurs d'un groupe -SH à chaque bout de sa chaîne, et se prêtant ainsi à des réactions intéressantes. Parmi les diènes les plus courants, justiciables du procédé de l'invention, on peut citer, à titre d'exemples les $\alpha$-$\omega$ dioléfines (non conjugués) comme pentadiène, hexadiène, octadiène, décadiène, undécadiène, dodécadiène, hexadécadiène, eicosadiène, etc.

Le procédé peut être exécuté en discontinu ou en continu, et se prête bien à ce dernier mode opératoire. Pour travailler en continu, on maintient sous pression, à la température voulue, un volant de $H_2S$ liquide dont on soutire continuellement une fraction qui est remplacée en même temps par de l'$H_2S$ frais et du diène frais. Ce débit est réglé de façon à ce que la teneur en diène, dans le milieu réactionnel, reste toujours entre les limites indiquées plus haut.

Les exemples, qui suivent, servent à illustrer l'invention et à la comparer avec la technique antérieure.

EXEMPLES 1 et 2

Les préparations suivantes ont été effectuées selon le procédé connu, décrit dans le brevet français publié sous le n° 2 501 679 : elles résidaient en la production d'hexanedithiol-1,6 à partir de l'hexadiène-1,5, en solution dans du benzène et de celle d'hexane-thiol à partir de l'héxène-1, également en solution benzénique. La réaction est initiée et entretenue par la lumière de longueur d'onde 350 nm, au sein de 200 ml de la solution benzénique renfermant par litre 0,033 mole de benzophénone (6g) et autant de moles de phosphite de triméthyle (4,1g).

La réaction est effectuée, comme dans les exemples 1 à 5 de FR 2 501 679 précité, dans un réacteur cylindrique en acier inoxydable, de 245 ml, équipé d'un tube coaxial en quartz, contenant une lampe UV, dont le maximum d'émission correspond à la longueur d'onde de 350 nm. La réfrigération et l'agitation du milieu réactionnel sont assurées par une boucle extérieure, constituée d'une pompe de recirculation et d'un échangeur permettant de maintenir la température du milieu réactionnel, à environ 10° C. On effectue deux séries d'essais dont les paramètres suivants sont maintenus fixes: débit d'$H_2S$ de 240 l/h, débit d'hydrocarbure 164 à 168 g/h, pression 12 bars. Le rapport molaire $H_2S$/hydrocarbure est maintenu à sensiblement 5.

Les conditions particulières à chaque essai et les résultats sont donnés dans le tableau suivant.

| EXEMPLE : | 1 | 2 |
|---|---|---|
| Hydrocarbure | Hexène-1 | Hexadiène-1,5 |
| Son débit en g/h | 168 | 164 |
| Thiol produit | Hexanethiol-1 | Hexanedithiol-1,6 |
| Production de thiol en g/h ........... | 127,5 | 75 |
| Conversion % de l'hydrocarbure ... | 77% | 90% |
| Rendement en thiol .... | 63% | 27% |
| Observations : | -- | Formation de sulfures lourds. |

On voit que la méthode classique, qui donne un bon résultat pour l'héxène-1 (exemple 1), est en défaut en ce qui concerne l'hexadiène-1,5 (exemple 2).
A noter également que le produit de réaction dans l'exemple 2 contient des polymères soufrés de structure $HS-(CH_2)_6-S-(CH_2)_6-S-(CH_2)_6$ insolubles dans le milieu réactionnel, qui forment une phase pâteuse.

EXEMPLE 3

Préparation de l'hexanedithiol-1,6 par l'action de l'$H_2S$ sur de l'hexadiène-1,5 en solution dans l'$H_2S$ liquide, sans autre solvant.

La réaction est effectuée dans un réacteur cylindrique en acier inoxydable de 300 ml, équipé d'un tube coaxial en quartz contenant une lampe, dontle maximum d'émission correspond à la longueur d'onde de 350 nm. Le refroidissement et l'agitation du milieu réactionnel sont assurés par une boucle extérieure, constituée d'une pompe de recirculation et d'un échangeur permettant de maintenir la température du milieu réactionnel au niveau désiré, 4°C. Le volume de l'ensemble réacteur et boucle est de 600 ml. La pression dans l'enceinte de réaction est réglée de telle sorte que l'hydrogène sulfuré soit liquide, 12 bars dans le cas présent. Avant d'allumer la lampe, on introduit dans le réacteur 300 ml d'$H_2S$ liquide. Puis on allume la lampe et, à l'aide d'une pompe, on introduit 240 ml d'hexadiène-1,5, contenant 3,6 g de benzophénone et 2,46g de phosphite de triméthyle, à raison de 200 ml à lheure. A cela s'ajoute le débit TPN d'hydrogène sulfuré gazeux de 200 litres/heures. La production est récupérée en continu.
On a obtenu 148 g d'hexane dithiol pur, soit un rendement de 48% sur la dioléfione mise en oeuvre, donc résultat presque double de celui de l'exemple 2 selon la technique antérieure.

EXEMPLE 4

Comme dans l'exemple 3, la préparation d'hexanedithiol-1,6 est effectuée en solution dans l'$H_2S$ liquide en excès, dans un réacteur de même capacité, mais de forme tubulaire, fonctionnant en écoulement dit "piston". Une double enveloppe extérieure, autour de ce tube, sert à éliminer la chaleur de la réaction.
A l'entrée du réacteur arrive, en continu, une solution à 9,1% en volume d'hexadiène-1,5 dans 300 ml de l'$H_2S$ liquide/heure. Cette solution contient 1,4% en poids de benzophénone et 2% de phosphite de tributyle par rapport à l'hexadiène.
Le débit total est de 330 ml/h, le temps de séjour dans le réacteur étant de 54 minutes. On opère à 10°C sous 14 bars. A la sortie du réacteur on trouve que 98% de l'hexadiène ont été convertis et de l'hexane dithiol-1,6 s'est formé avec une sélectivité de 63% et une production horaire de 23,8g.

EXEMPLE 5

Une préparation semblable à celle de l'exemple 4 est conduite avec la seule différence que les débits sont doublés : $H_2S$ 600 ml/h, hexadiène 60 ml/h. La conversion de l'hexadiène est alors de 91%, et la sélectivité en hexane dithiol de 60%, avec une production horaire de 42,1g.

EXEMPLE 6

Le mode opératoire est le même qu'à l'exemple 4, sauf une légère différence du débit total, 315 ml/h au lieu de 330.
Mais la solution d'alimentation ne titre que 5% d'hexadiène dans l'$H_2S$ liquide, contre 9,1% dans l'exemple 4.
La conversion de l'hexadiène est alors de 98%, la sélectivité en dithiol 68% et la production de celui-ci de 10,7 g/h.
La comparaison de cet essai avec celui de l'exemple 4 montre que l'abaissement de la concentration en diène de 9,1 à 5% se traduit par une amélioration de la sélectivité en dithiol de 63 à 68%.

EXEMPLE 7

On a opéré comme dans l'exemple 4, mais avec une solution d'alimentation en hexadiène-1,5 à 2,5% seulement, le débit total étant de 620 ml/h. La conversion du diène était alors de 100%, la sélectivité en dithiol 71% et la production horaire de celui-ci 11,9 g.
On voit ainsi que la sélectivité est d'autant meilleure que la concentration en diène est plus faible dans le milieu réactionnel.

EXEMPLE 8

En suivant la technique de l'exemple 5, on a remplacé l'hexadiène par du décadiène-1,9, sous la forme d'une solution à 10% dans de l'$H_2S$. Le débit total était de 660 ml/h. La conversion du décadiène était de 91% et la sélectivité en décane dithiol-1,10 de 63% avec une production de ce dernier de 39,5 g/h.

EXEMPLE 9

Les opérations de l'exemple 8 sont répétées avec 7% en volume de diène dans le milieu réactionnel.
Les diènes suivants sont essayés : hexadiène-1,4 ; heptadiène-1,4; octadiènes-1,7 et 1,4 ; décadiène-1,9 ; dodécadiène-1,11 ; octadécadiène-6,9 ; eicosadiène-1,19.
Dans chaque cas on obtient le dithiol correspondant avec une sélectivité intermédiaire entre celles des exemples 5 et 7.

EXEMPLE 10

L'exemple 6 est reproduit, mais avec de la thioxanthone à la place de la benzophénone. Une sélectivité de 70% en dithiol est obtenue.

**Revendications**

1. Procédé de production de dithiol par l'action de l'hydrogène sulfuré sur un diène non conjugué en présence de la lumière de longueur d'onde de 300 à 600 nm et d'un système catalytique comprenant une cétone ou thiocétone aromatique et un phosphite trialkylique, caractérisé en ce que le diène est dissous ou dispersé dans de l'$H_2S$ liquide présent en excès et que la teneur volumétrique en diène, dans le mélange réactionnel, est de 2,5 à 10%.

2. Procédé selon la revendication 1 , caractérisé en ce que le dithiol formé est retiré du milieu réactionnel de façon à ce que son accumulation dans le milieu ne dépasse pas 10%.

3. Procédé selon la revendication 2, caractérisé en ce que, la production étant continue, le dithiol est retiré du milieu réactionnel au fur et à mesure de sa formation.

4. Procédé selon l'une des revendications précédentes, caractérisé en ce que les composés constituant le système catalytique sont présents à raison de 0,01 à 0,1 mole, chacun, par litre de milieu réactionnel.

5. Procédé selon l'une des revendications précédentes, caractérisé en ce que le diène utilisé est en $C_5$ à $C_{20}$.

6. Procédé selon la revendication 5 , caractérisé en ce que le diène est α-ω.

7. Procédé selon l'une des revendications précédentes, caractérisé en ce que la réaction a lieu à une température de -10° à +50° C et de préférence entre -5° et +15° C, sous la pression de vapeur de l'$H_2S$ correspondante.

**Claims**

1. Process for the production of dithiol by the action of hydrogen sulphide on an unconjugated diene in the presence of light of wavelength from 300 to 600 nm and of a catalyst system comprising an aromatic ketone or thioketone and a trialkyl phosphite, characterised in that the diene is dissolved or dispersed in liquid $H_2S$ present in excess and that the volumetric concentration of diene in the reaction mixture is from 2.5 to 10%.

2. Process according to Claim 1, characterised in that the dithiol formed is withdrawn from the reaction mixture so that its accumulation in the mixture does not exceed 10%.

3. Process according to Claim 2, characterised in that, the production being continuous, the dithiol is withdrawn from the reaction mixture as it is being formed.

4. Process according to one of the preceding claims, characterised in that the compounds forming the catalyst system are present in a proportion of 0.01 to 0.1 mole of each per litre of reaction mixture.

5. Process according to one of the preceding claims, characterised in that the diene employed is $C_5$ - $C_{20}$.

6. Process according to Claim 5, characterised in that the diene is α-ω.

7. Process according to one of the preceding claims, characterised in that the reaction takes place at a temperature of -10° to +50° C and preferably between -5° and +15° C, at the corresponding vapour pressure of $H_2S$.

**Patentansprüche**

1. Verfahren zur Herstellung von Dithiol durch Einwirkung von Schwefelwasserstoff auf ein nicht konjugiertes Dien in Gegenwart von Licht einer Wellenlänge von 300 bis 600 nm und einem katalytischen System, das ein aromatisches Keton oder Thioketon und ein Trialkylphosphit umfaßt, dadurch gekennzeichnet, daß das Dien in dem im Überschuß vorliegenden flüssigen $H_2S$ gelöst oder dispergiert ist und daß der Dienanteil im Reaktionsgemisch 2,5 bis 10 Vol.% beträgt.

2. Verfahren nach Anspruch 1, dadurch gekennzeichnet, daß das gebildete Dithiol derart vom Reaktionsmedium abgezogen wird, daß seine Anreicherung im Medium 10% nicht übersteigt.

3. Verfahren nach Anspruch 2, dadurch gekennzeichnet, daß das Dithiol bei kontinuierlicher Herstellung dem Ausmaß seiner Bildung entsprechend vom Reaktionsmedium abgezogen wird.

4. Verfahren nach einem der vorhergehenden Ansprüche, dadurch gekennzeichnet, daß die das katalytische System bildenden Verbindungen jeweils in einem Anteil von 0,01 bis 0,1 mol pro Liter des Reaktionsmediums vorhanden sind.

5. Verfahren nach einem der vorhergehenden Ansprüche, dadurch gekennzeichnet, daß Diene mit 5 bis 20 Kohlenstoffatomen verwendet werden.

6. Verfahren nach Anspruch 5, dadurch gekennzeichnet, daß es ein α-ω-Dien ist.

7. Verfahren nach einem der vorhergehenden Ansprüche, dadurch gekennzeichnet, daß die Reaktion bei einer Temperatur von - 10 bis + 50° C und vorzugsweise zwischen - 5 und + 15° C unter dem korrespondierenden $H_2S$-Dampfdruck stattfindet.